# FASCICULE DE BREVET EUROPEEN

(11) **EP 2 574 361 B1**
(45) Date de publication et mention de la délivrance du brevet: **02.10.2019**
(21) Numéro de dépôt: 12179651.0
(22) Date de dépôt: 08.08.2012
(51) Int. Cl.: A61M 16/10, A62B 25/00, F17C 13/08, A61M 16/00

(54) **Chariot de transport d'une bouteille de gaz médical**
Transportkarren für eine medizinische Gasflasche
Cart for transporting a medical gas cylinder

(30) Priorité: 30.09.2011 FR 1158811
(43) Date de publication de la demande: 03.04.2013
(73) Titulaire: Air Liquide Santé (International), 75007 Paris (FR); L'AIR LIQUIDE, SOCIETE ANONYME POUR L'ETUDE ET L'EXPLOITATION DES PROCEDES GEORGES CLAUDE, 75007 Paris (FR); Air Liquide Santé France, 75007 Paris (FR)
(72) Inventeur: Dori, Maxime, 31750 Escalquens (FR); Femy, Marianne, 75013 Paris (FR)
(74) Mandataire: Pittis, Olivier

(56) Documents cités:
- FR-A1- 2 910 362
- FR-A1- 2 926 728
- US-A1- 2009 121 592
- US-A1- 2011 203 587
- KERSTEN J: "DAS NEUE MEDIMORPH-SYSTEM", MEDIZINTECHNIK, KUNST UND WISSEN ERICH BIEBER, ZUERICH, CH, vol. 26, no. 3, 1 septembre 1986 (1986-09-01), pages 66-70, XP000006531,

## Description

L'invention porte sur un chariot de soins mobile, tel un chariot de sédation, servant à transporter une bouteille de gaz médical et un ou des appareils médicaux, tel les dispositifs d'administration et/ou un oxymètre, lequel chariot est particulièrement bien adapté à une utilisation en milieu médical ou hospitalier ou encore en cabinet de soins, par exemple en cabinet dentaire.

Certains gaz médicaux, tels les mélanges de sédation formés d'une proportion équimolaire (50%/50%) de N₂O et d'oxygène sont utilisés tant à l'hôpital que dans les cabinets médicaux, en particulier les cabinets dentaires, où ils servent à soulager ou à atténuer la douleur chez des patients ayant à subir une intervention médicale légère, comme une extraction de dent par exemple.

Ces gaz sont conditionnés en bouteille de gaz de contenant typique de l'ordre de 5 litres (contenance en équivalent eau) qui pèsent plusieurs kilogrammes et ne sont donc pas forcément faciles à manipuler.

En outre, l'administration du gaz requiert l'usage de matériels spécifiques, tel un kit d'administration comprenant tuyauterie, ballon d'insufflation et masque respiratoire, voire d'un appareil médical d'administration de gaz.

Or, ces matériels sont encombrants et peu pratiques à ranger et à manipuler pour le personnel soignant.

Des appareils de l'état de la technique sont décrits dans les documents
- "DAS NEUE MEDIMORPH-SYSTEM" publié le 01-09-1986 par KERSTEN J, dans le journal MEDIZINTECHNIK, KUNST UND WISSEN ERICH BIEBER, ZUERICH, CH, Volume 26, numéro 3, pages 66-70,
   US2009121592 A1,
   FR2926728 A1,
   FR2910362 A1, et
   US2011203587 A1.

Au vu de cela, le problème qui se pose est de faciliter la manipulation et la mise en oeuvre des bouteilles de gaz médical et des instruments ou matériels associés, tel qu'un oxymètre, dans les bâtiments hospitaliers ou les cabinets médicaux, tout en permettant un accès à, une visualisation et/ou un réglage aisés et simples des organes de réglage du robinet ou robinet-détendeur de distribution de gaz équipant les bouteilles de gaz.

La solution est un chariot ergonomique mobile selon la revendication 1.

Selon le cas, le chariot peut comprendre l'une ou plusieurs des caractéristiques techniques suivantes :
- un compartiment de rangement est agencé sous la structure plateau, ledit compartiment comprenant une ou plusieurs ouvertures donnant accès à un espace interne, c'est-à-dire un espace de rangement pour de petits matériels.
- les deux bras-porteurs sont fixés par leurs secondes extrémités directement à la structure plateau ou indirectement à ladite structure plateau par l'intermédiaire du compartiment de rangement agencé sous ladite structure plateau.
- lesdits bras-porteurs latéraux ont une forme longiligne incurvée et dirigée vers la face arrière de la paroi périphérique. En effet, grâce à cette forme incurvée vers l'arrière des bras-porteurs ou potences de portage du plateau, l'utilisateur a une bonne visibilité et peut opérer un réglage aisé du débit de gaz sortant de la bouteille, via l'organe de réglage rotatif, tel un volant rotatif, du robinet-détendeur qui est monté sur la bouteille et qui est accessible par l'intermédiaire d'une ouverture située en partie haute du chapeau de protection équipant la bouteille.
- la structure plateau est plane et horizontale ou quasi-horizontale, lorsque les roues du chariot sont posées sur un sol horizontal ou quasi-horizontal.
- la structure plateau comprend une poignée.
- la structure plateau comprend une poignée en forme de fente aménagée dans ladite structure plateau et dimensionnée pour permettre l'insertion dans ladite fente de plusieurs doigts d'une main de manière à permettre à un opérateur d'agripper le chariot par ladite fente.
- l'embase, le fond et la paroi sont moulés d'une seule pièce, de préférence ils sont en matériau polymère.
- les deux bras-porteurs et le plateau sont également en matériau polymère.
- les deux bras-porteurs viennent se raccorder à la paroi au niveau de deux expansions de paroi faisant saillie vers le haut, de préférence lesdites expansions de paroi sont agencées dans les deux faces latérales, en particulier les expansions de paroi sont formée d'une partie de la paroi desdites deux faces latérales.
- les bras-porteurs sont maintenus solidaires de la paroi de l'embase grâce à des éléments de fixation, de préférence les éléments de fixation sont des éléments se fixant par vissage, tel que des vis, des boulons ou analogues.
- les bras-porteurs viennent s'emboiter dans ou se fixer à l'embase au niveau des expansions de paroi.
- les bras-porteurs et les expansions de paroi présentent des structures tridimensionnelles complémentaires de manière à permettre leur couplage solidaire, c'est-à-dire leur emboitement et maintien solidaire les uns dans les autres.
- les bras-porteurs et les expansions de paroi comprennent des rails de guidage et glissières coopérant ensemble pour assurer le montage desdits bras-porteurs sur les expansions de paroi
- les bras-porteurs sont fixés aux expansions de paroi par le biais d'une pièce intermédiaire de maintien.
- la structure plateau comprend en outre au moins un dispositif d'accrochage, de préférence plusieurs dispositifs d'accrochage.
- au moins l'un des dispositifs d'accrochage est adapté et conçu pour l'accroche d'un ballon respiratoire notamment.
- la structure plateau comprend au moins deux dispositifs d'accrochage comprenant chacun une ou plusieurs encoches aménagées dans le plateau, de préférence des encoches de dimensions différents pouvant accueillir plusieurs types de kits d'administration différents. Préférentiellement, au total, le plateau comporte 4 encoches, deux de chaque cotés du plateau pour une utilisation facilitée aussi bien pour les droitiers que pour les gauchers.
- la bouteille de gaz contient un gaz médical choisi parmi N₂O, O₂ et les mélanges O₂/N₂O.
- il peut supporter un appareil médical agencé sur la structure-plateau, en particulier un oxymètre ou un appareil respiratoire relié à la bouteille de gaz et à un masque respiratoire, via des canalisations de gaz, de préférence des canalisations flexibles.

L'invention va être mieux comprise grâce à la description détaillée suivante faite en références aux Figures annexées parmi lesquelles :
- la Figure 1 représente une vue de devant d'un chariot mobile de soins selon un mode de réalisation conforme à la présente invention, sans bouteille insérée,
- la Figure 2 représente le chariot mobile de la Figure 1 vu de derrière,
- la Figure 3 représente le chariot mobile de la Figure 1 avec bouteille insérée dans le logement central et équipé d'instruments médicaux, et
- les Figures 4 et 5 détaillent le système de fixation des bras-porteurs à l'embase du chariot des Figures 1 à 3.

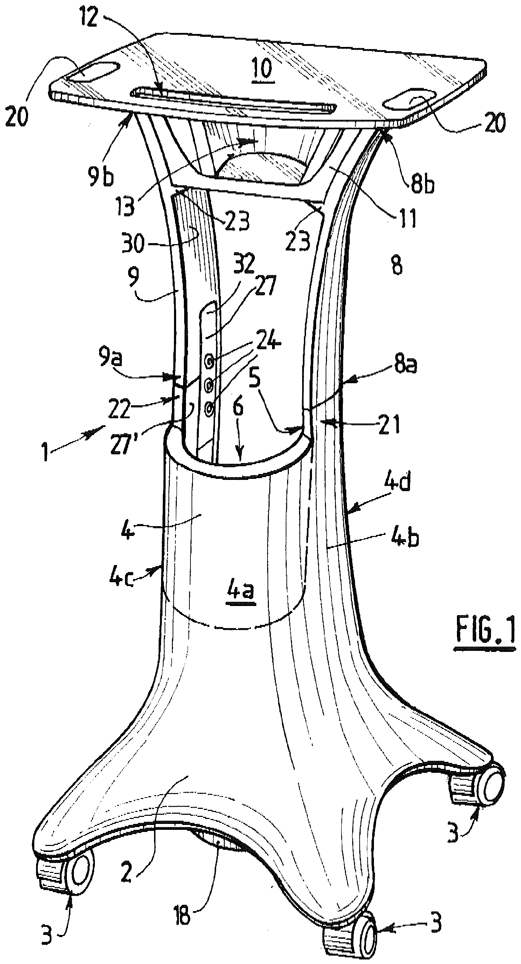

La Figure 1 représente un mode de réalisation d'un chariot 1 mobile de soins selon la présente invention particulièrement adapté à une utilisation en milieu hospitalier ou en cabinet de soins, par exemple en cabinet dentaire, pour transporter une bouteille de gaz 17, ainsi que différents matériels de soins, notamment instruments divers 25, tel des masques respiratoires, appareil respiratoire ou oxymètre 33, ballon d'insufflation 14, tuyauteries 26...

Ce chariot 1 comprend une partie basse ou embase 2 qui est munie d'un fond et de plusieurs roues 3, typiquement de 3 à 5 roues, permettant d'assurer les déplacements du chariot sur le sol.

De façon optionnelle, les roues 3 peuvent être équipées d'un système de blocage empêchant leur libre rotation de manière à bloquer le chariot 1 et à empêcher ses déplacements sur le sol. Toutefois, la présence de freins n'est pas obligatoire et le chariot 1 des Figures 1 à 3 n'en comporte pas.

L'embase 2 est surmontée d'une paroi périphérique 4 se projetant vers le haut et conformée de manière à délimiter un volume interne 6 dimensionné pour former un logement apte à recevoir une bouteille de gaz 7 de forme cylindrique. Plus précisément, la paroi 4 comporte une face avant 4a et deux faces latérales droite et gauche 4b, 4c fermées. Elle forme donc une sorte de coque protectrice périphérique protégeant la majeure partie du fût de la bouteille 7 et l'empêchant de basculer vers l'avant et les côtés.

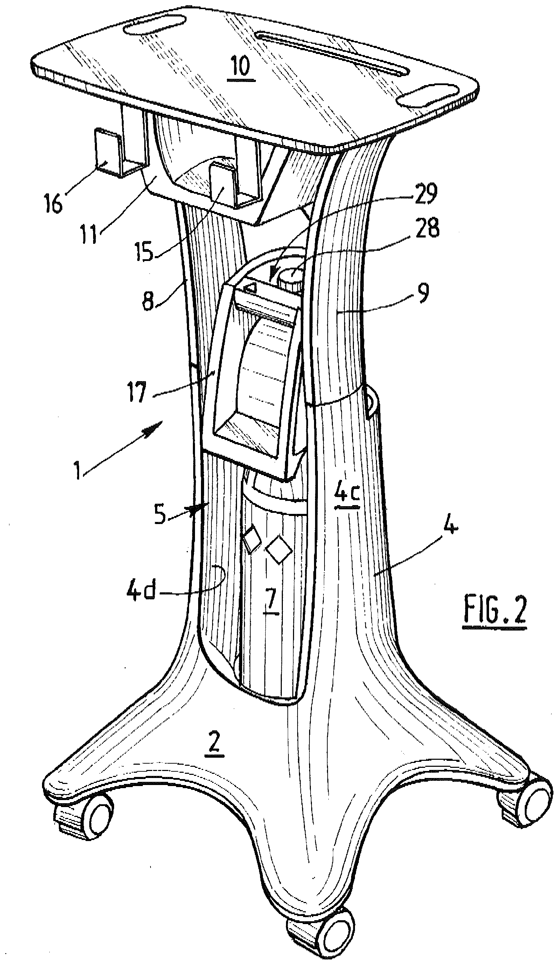

En outre, comme visible sur la Figure 2, une ouverture 5 est pratiquée dans la face arrière 4d de la paroi 4 pour permettre une insertion facile de la bouteille de gaz 7 dans le volume interne 6, y compris lorsque la bouteille 7 comporte un capotage ou chapeau 17 de protection servant à protéger le robinet-détendeur 18 et les autres éléments servant à la distribution du gaz, notamment un manomètre 19 et un volant de réglage 28. Une telle architecture de bouteille est connue du document EP-A-629812.

Comme on le comprend au vu de la Figure 2, la face avant 4a et les deux faces latérales 4b, 4c du chariot forme un compartiment ayant une section en U ou quasi en U, considérée au niveau de l'ouverture 5, et formant la coque protectrice périphérique susmentionné.

Lorsqu'elle est insérée dans le volume interne 6 du chariot 1, via l'ouverture 5 aménagée dans sa face arrière 4d, la bouteille de gaz 7 repose sur le fond de l'embase 2.

Le fond (non visible sur les Figures) de l'embase 2 peut être conformé pour recevoir, voire bloquer, la partie basse du fût d'une bouteille 7 de gaz. Par exemple, il peut comprendre une zone en retrait 18 faisant saillie vers le dessous du chariot, c'est-à-dire vers le sol, et conformée pour présenter une forme tridimensionnelle cylindrique apte à recevoir la partie basse du fût de la bouteille 7, tel un manchon. Une telle configuration du fond de l'embase 2 permet de mieux maintenir le fût de la bouteille 17 dans le logement 6 et d'éviter un basculement de la bouteille 7 par l'ouverture 5 arrière aménagée dans la paroi arrière 4d. Optionnellement, une sangle de maintien ou analogue peut être prévue au niveau de l'ouverture 5 pratiquée dans la paroi arrière 4d.

Le volume interne 6 formant logement central pour la bouteille 7 est conçu et dimensionné pour recevoir des bouteilles de gaz typiquement de type B5 d'environ 70 cm de hauteur (chapeau 17 compris) et de 15 cm de diamètre au niveau du fût de la bouteille. Toutefois, le volume interne peut être adapté à la réception de bouteilles de dimensions différentes.

Par ailleurs, deux bras-porteurs 8, 9 surmontés d'un plateau 10 sont fixés aux deux faces latérales droite et gauche 4b, 4c de la paroi 4.

Plus précisément, le chariot 1 est formé de deux sous-unités venant se connecter l'une à l'autre comprenant une première sous-unité (formant la partie basse du chariot) comprenant l'embase 2, les roues 3, le fond et la paroi 4, et une deuxième sous-unité (formant la partie haute du chariot) comprenant les bras-porteurs 8, 9 et la structure-plateau, et éventuellement le compartiment 11, notamment lorsque celui-ci est formé d'une seule pièce avec les bras 8, 9 et le plateau 10, ou y est fixer solidairement.

En fait, les deux bras-porteurs 8, 9 viennent se raccorder, via leurs premières extrémités 8a, 9a, à la paroi 4 au niveau de deux expansions de paroi 21, 22 faisant saillie vers le haut à partir des deux faces latérales 4b, 4c de ladite paroi 4.

Les bras-porteurs 8, 9 sont ensuite maintenus solidaires de la paroi 4 de l'embase 2 grâce à des éléments de fixation 24, telles des vis ou tout élément analogue à fixation par vissage.

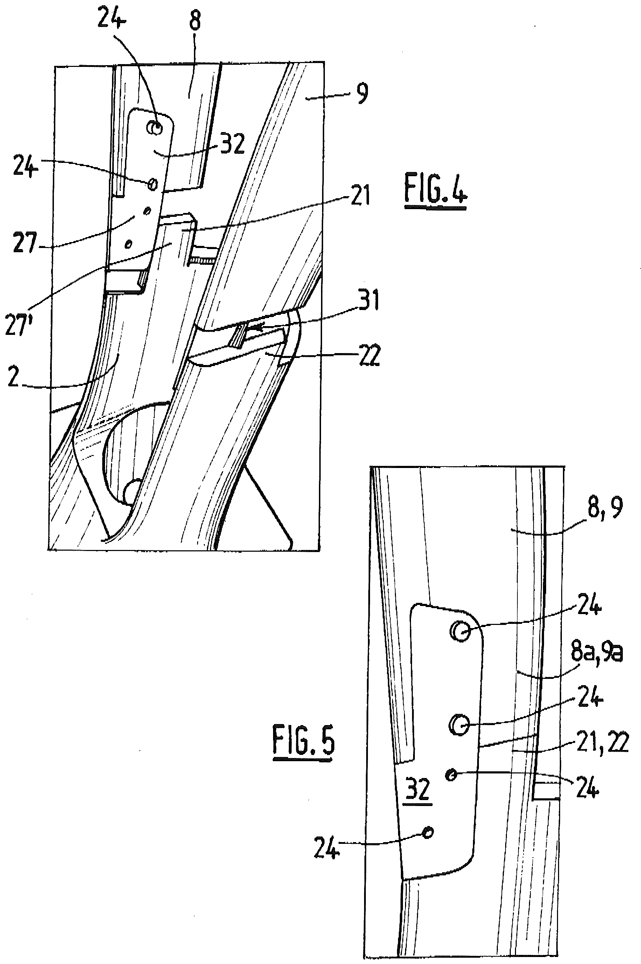

Comme détaillé sur les Figures 4 et 5, les bras-porteurs 8, 9 viennent en fait s'emboiter dans l'embase 2 au niveau des expansions de paroi 21, 22 de la paroi 4.

Pour assurer un emboitement adéquat et efficace, les bras-porteurs 8, 9 et les expansions de paroi 21, 22 présentant des structures tridimensionnelles 27, 27' complémentaires, de forme plus ou moins complexe, de manière à permettre leur couplage et leur maintien en position les uns dans les autres.

Selon le mode de réalisation considéré, les bras-porteurs 8, 9 et les expansions de paroi 21, 22 peuvent présenter des structures tridimensionnelles 27, 27' complémentaires venant s'emboiter et/ou se coupler les unes aux autres, en étant maintenues fixées ensemble via des éléments de fixation 24, telles de vis, des boulons ou analogues.

Ces structures tridimensionnelles 27, 27' complémentaires permettent non seulement de faciliter le montage des deux sous-unités mais aussi d'éviter les erreurs de montage car, en cas d'inversion de sens, le montage des sous-unités ne peut pas se faire.

Afin de faciliter l'opération de montage, les bras-porteurs 8, 9 et/ou les expansions de paroi 21, 22 peuvent comprendre des rails de guidage 31 coopérant avec des glissières en venant s'insérer les uns dans les autres et faciliter ainsi l'emboitement des sous-unités entre elles.

Les éléments de fixation 24 notamment par vissage, telles des vis ou analogue, peuvent raccorder directement les bras-porteurs 8, 9 aux expansions de paroi 21, 22 ou alors indirectement par le biais d'une pièce intermédiaire de maintien 32, comme visible sur les Figures 4 et 5, laquelle vient se fixer aux bras-porteurs 8, 9 et aux expansions de paroi 21, 22.

Chaque bras-porteur 8, 9 se projette donc vers le haut à partir de sa première extrémité 8a, 9a qui est fixée à l'une des deux expansions de paroi 21, 22 situées en haut des faces latérales 4b, 4c de la paroi périphérique 4, et soutient via sa seconde extrémité 8b, 9b la structure-plateau 10 surmontant le chariot 1.

Cette structure-plateau 10 est sensiblement parallèle au fond du chariot et à la surface du sol sur laquelle repose le chariot 1.

Comme visible sur la Figure 1, un compartiment 11 ou bac de rangement est agencé juste en dessous de la structure plateau 10, lequel comprend une ouverture avant 13 donnant accès à l'espace interne du compartiment 11. Il est préférablement constitué d'un polymère.

Le plateau 10 forme le toit ou paroi supérieure du compartiment 11.

Par ailleurs, afin de permettre une évacuation de liquide qui pourrait s'accumuler dans le compartiment 11, celui-ci peut comprendre un ou des orifices d'évacuation percés dans son fond.

Le compartiment 11 est fixé aux bras 8, 9 par exemple par vissage ou alors par simple clipsage en étant maintenu en position in situ via des épaulements 23 se faisant face, agencés sur la paroi interne 30 de chacun des bras 8, 9, comme illustré en Figure 1. Ces épaulements 23 font donc saillie l'un vers l'autre et le compartiment 11 vient reposer dessus en étant maintenu bloqué entre lesdits épaulements 23 et le plateau 10.

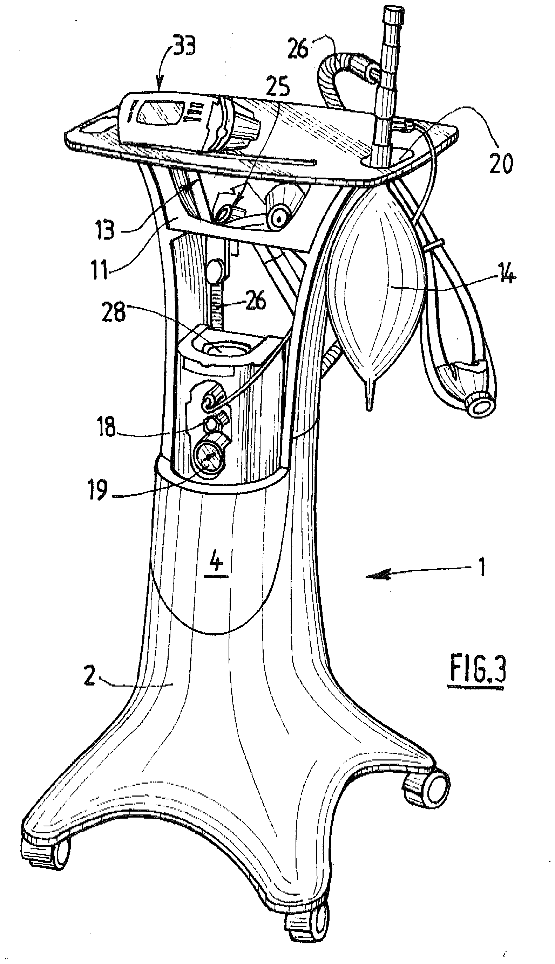

Comme illustré sur la Figure 3, le compartiment 11 et le plateau 10 permettent de recevoir des ustensiles 25, des dispositifs ou appareils médicaux, tel des appareils d'oxymétrie ou d'administration de gaz médical, ainsi que leur tuyauterie flexible 26 ou leurs câbles de connexion électrique, et/ou des instruments chirurgicaux ou de soins susceptibles d'être utilisés par le personnel soignant.

La structure plateau 10 comprend en outre une poignée 12 se présentant sous forme d'une fente aménagée dans la structure plateau 10 et dimensionnée pour permettre l'insertion dans celle-ci de plusieurs doigts de la main d'un utilisateur de manière à permettre à l'utilisateur d'agripper le chariot 1 par cette fente et donc de faciliter ainsi son orientation ou son déplacement sur le sol.

Il est à noter que, selon le mode de réalisation choisi, les deux bras-porteurs 8, 9 peuvent être fixés par leurs secondes extrémités 8b, 9b directement à la structure plateau 10 ou, selon le cas, indirectement à celle-ci, c'est-à-dire par exemple par l'intermédiaire du compartiment 11 de rangement agencé sous ladite structure plateau 10.

De préférence, l'embase 2, le fond, la paroi 4 et les bras-porteurs 8, 9 sont moulés d'une seule pièce, de préférence ils sont en matériau polymère.

Par ailleurs, comme illustré en Figure 2, le plateau 10 ou la face arrière du compartiment 11 peuvent être dotés d'un ou plusieurs crochets 15, 16 servant à accrocher des instruments servant pendant les soins, tels des tuyaux ou conduites souples.

Comme visible en Figures 1 et 2, les bras-porteurs latéraux 8, 9 ont une forme longiligne incurvée et dirigée vers la face arrière 4d de la paroi périphérique 4. Ils sont de préférence de forme aplatie, c'est-à-dire formés d'une plaque. En effet, grâce à cette forme incurvée vers l'arrière des bras-porteurs 8, 9, encore appelés potences de portage, le plateau 10 est décalé vers l'arrière du chariot 1, et non pas à l'aplomb de la bouteille 7, et dès lors l'utilisateur a une bonne visibilité et peut opérer un réglage aisé du débit de gaz sortant de la bouteille 7, via l'organe de réglage 28 rotatif, tel un volant rotatif, du robinet-détendeur qui est monté sur la bouteille et qui est accessible par l'intermédiaire d'une ouverture 29 située en partie haute du chapeau de protection 17 équipant la bouteille 7.

La structure plateau 10 comprend en outre un dispositif d'accrochage 20 adapté et conçu pour permettre la fixation d'un ballon respiratoire 14. Le dispositif d'accrochage 20 comprend des encoches aménagées dans le plateau 10 ayant de préférence des dimensions différentes de sorte de pouvoir recevoir plusieurs types de kits d'administration différents. Préférentiellement, le plateau comporte 4 encoches ou analogues, deux de chaque cotés du plateau 10 pour une utilisation facilitée aussi bien pour les droitiers que pour les gauchers.

La bouteille de gaz 7 positionnée au sein du logement 5 contient un gaz médical à un ou plusieurs composés gazeux, par exemple un mélange équimolaire (50/50%) de N₂O et d'oxygène.

Lorsque la bouteille 7 est insérée dans le logement du chariot 1, la paroi 4 peut recouvrir totalement le corps de la bouteille 7, à l'exception du chapeau 17, comme visible sur le mode de réalisation de la Figure 3, ou alors seulement une partie du corps de bouteille selon le mode de réalisation choisi.

Le poids du chariot 1 de l'invention est de l'ordre de 14 kg, sa hauteur de l'ordre de 97 cm et sa surface au sol de 45 cm x 45 cm.

Tous les polymères, en particulier les plastiques, entrant dans la constitution du chariot 1 de l'invention doivent être compatibles avec un nettoyage par des produits désinfectants classiques, y compris des solvants.

Le chariot 1 selon l'invention est utilisable au sein d'un bâtiment hospitalier pour transporter au moins une bouteille 7 de gaz, et éventuellement un appareil médical agencé sur la structure-plateau 10, en particulier un oxymètre 33, ainsi que d'autres matériels, notamment un ballon d'insufflation 14.

Le chariot 1 de l'invention est particulièrement adapté à une utilisation en cabinet de dentiste ou de dermatologie.

## Revendications

1. Chariot (1) mobile comprenant :
- une embase (2) avec un fond et munie de plusieurs roues (3),
- l'embase (2) étant surmontée d'une paroi périphérique (4) se projetant vers le haut et conformée de manière à délimiter un volume interne (6) dimensionné pour former un logement apte à recevoir une bouteille de gaz (7) de forme cylindrique, ladite bouteille de gaz (7) reposant sur ledit fond, et
- deux bras-porteurs (8, 9) se projetant vers le haut, les bras-porteur (8, 9) étant fixés par une première extrémité (8a, 9a) à l'une des faces latérales (4b, 4c) de la paroi périphérique (4) et par une seconde extrémité (8b, 9b) à une structure plateau (10) surmontant le chariot (1),
**caractérisé en ce que** :
- ladite paroi périphérique (4) comprenant une face avant (4a) située à l'opposé à la face arrière (4d) munie de l'ouverture (5), et deux faces latérales (4b, 4c) située à l'opposé l'une de l'autre, par rapport au volume interne (6), la face avant (4a) et les deux faces latérales (4b, 4c) formant un compartiment pour bouteille de volume interne (6) et de section en U ou quasi en U, considérée au niveau de l'ouverture (5), la paroi périphérique (4) présentant une ouverture (5) aménagée dans la face arrière (4d) permettant l'insertion d'une bouteille (7) de gaz au sein dudit volume interne (6) et son positionnement sur le fond de l'embase (2), et
- le chariot (1) est formé de 2 sous-unités venant se connecter l'une à l'autre comprenant :
. une première sous-unité comprenant au moins l'embase (2), les roues (3), le fond de l'embase (2) et la paroi (4) périphérique, et
. une deuxième sous-unité comprenant au moins les bras-porteurs (8, 9) et la structure-plateau (10).

2. Chariot selon la revendication précédente, **caractérisé en ce qu'**un compartiment (11) de rangement est agencé sous la structure plateau (10), ledit compartiment (11) comprend une ou plusieurs ouvertures (13) donnant accès à un espace interne.

3. Chariot selon l'une des revendications précédentes, **caractérisé en ce que** la structure plateau (10) est plane et horizontale ou quasi-horizontale, lorsque les roues du chariot sont posées sur un sol horizontal ou quasi-horizontal.

4. Chariot selon l'une des revendications précédentes, **caractérisé en ce que** lesdits bras-porteurs latéraux (8, 9) ont une forme longiligne incurvée et dirigée vers la face arrière (4d) de la paroi périphérique (4).

5. Chariot selon l'une des revendications précédentes, **caractérisé en ce que** la structure plateau (10) comprend une poignée (12) en forme de fente aménagée dans ladite structure plateau (10) et dimensionnée pour permettre l'insertion dans ladite fente de plusieurs doigts d'une main de manière à permettre à un opérateur d'agripper le chariot (1) par ladite fente.

6. Chariot selon l'une des revendications précédentes, **caractérisé en ce que** les deux bras-porteurs (8, 9) viennent se raccorder à la paroi (4) au niveau de deux expansions de paroi (21, 22) faisant saillie vers le haut, de préférence lesdites expansions de paroi (21, 22) sont agencées dans les deux faces latérales (4b, 4c).

7. Chariot selon la revendication 6, **caractérisé en ce que** les bras-porteurs (8, 9) viennent s'emboiter dans ou se fixer à l'embase (2) au niveau des expansions de paroi (21, 22), de préférence lesdits bras-porteurs (8, 9) et lesdites expansions de paroi (21, 22) présentent des structures tridimensionnelles complémentaires (27, 27').

8. Chariot selon l'une des revendications 6 ou 7, **caractérisé en ce que** les bras-porteurs (8, 9) et les expansions de paroi (21, 22) comprennent des rails de guidage (31) et glissières coopérant ensemble pour assurer le montage desdits bras-porteurs (8, 9) sur les expansions de paroi (21, 22)

9. Chariot selon l'une des revendications 6, 7 ou 8, **caractérisé en ce que** les bras-porteurs (8, 9) sont fixés aux expansions de paroi (21, 22) par le biais d'une pièce intermédiaire de maintien (32).

10. Chariot selon l'une des revendications précédentes, **caractérisé en ce que** la structure plateau (10) comprend en outre au moins un dispositif d'accrochage (20), de préférence plusieurs dispositifs d'accrochage, notamment adapté et conçu pour l'accroche d'un ballon respiratoire (14).

11. Chariot selon l'une des revendications précédentes, **caractérisé en ce que** l'embase (2), le fond, la paroi (4) sont moulés d'une seule pièce, de préférence ils sont en matériau polymère.

12. Chariot selon l'une des revendications précédentes, **caractérisé en ce qu'**il comporte un dispositif de fixation (18) apte à et conçu pour maintenir la bouteille de gaz (7) au sein du logement (5), en particulier une bouteille d'un gaz médical choisi parmi N₂O, O₂ et les mélanges O₂/N₂O..

13. Utilisation d'un chariot (1) selon l'une des revendications précédentes au sein d'un bâtiment hospitalier pour transporter au moins une bouteille de gaz, et éventuellement un appareil médical (25) agencé sur la structure-plateau (10), en particulier un oxymètre.

## Patentansprüche

1. Beweglicher Karren (1), umfassend:
- einen Sockel (2) mit einem Boden und ausgestattet mit mehreren Rädern (3),
- wobei der Sockel (2) von einer Umfangswand (4) überragt ist, die nach oben vorspringt und ausgerichtet ist, um ein inneres Volumen (6) zu begrenzen, das abgemessen ist, um eine Aufnahme zu bilden, die ausgelegt ist, um eine Gasflasche (7) mit zylindrischer Form aufzunehmen, wobei die Gasflasche (7) auf dem Boden aufliegt, und
- zwei Tragarme (8, 9), die nach oben vorspringen, wobei die Tragarme (8, 9) durch ein erstes Ende (8a, 9a) an einer der lateralen Seiten (4b, 4c) der Umfangswand (4) und durch ein zweites Ende (8b, 9b) an einer Tablettstruktur (10) befestigt sind, die den Karren (1) überragt,
**dadurch gekennzeichnet, dass**:
- die Umfangswand (4) eine Vorderseite (4a) umfasst, die sich gegenüber der Rückseite (4d), ausgestattet mit der Öffnung (5), befindet, und zwei laterale Seiten (4b, 4c), die sich einander gegenüber mit Bezug auf das innere Volumen (6) befinden, wobei die Vorderseite (4a) und die zwei lateralen Seiten (4b, 4c) ein Fach für eine Flasche mit einem inneren Volumen (6) und einem U-förmigen oder fast U-förmigen Schnitt, betrachtet auf der Ebene der Öffnung (5), bilden, wobei die Umfangswand (4) eine Öffnung (5) aufweist, die in der Rückseite (4d) angebracht ist, die die Einführung einer Gasflasche (7) in das innere Volumen (6) und ihre Positionierung auf dem Boden des Sockels (2) ermöglicht, und
- der Karren (1) aus 2 Untereinheiten gebildet ist, die sich untereinander verbinden, umfassend:
. eine erste Untereinheit, umfassend mindestens den Sockel (2), die Räder (3), den Boden des Sockels (2) und die Umfangswand (4), und
. eine zweite Untereinheit, umfassend mindestens die Tragarme (8, 9) und die Tablettstruktur (10).

2. Karren nach dem vorstehenden Anspruch, **dadurch gekennzeichnet, dass** ein Ablagefach (11) unter der Tablettstruktur (10) angeordnet ist, wobei das Fach (11) eine oder mehrere Öffnungen (13) umfasst, die Zugang zu einem inneren Raum gewähren.

3. Karren nach einem der vorstehenden Ansprüche, **dadurch gekennzeichnet, dass** die Tablettstruktur (10) eben und horizontal oder fast horizontal ist, wenn die Räder des Karrens auf einen horizontalen oder fast horizontalen Boden gestellt sind.

4. Karren nach einem der vorstehenden Ansprüche, **dadurch gekennzeichnet, dass** die lateralen Tragarme (8, 9) eine langgezogene gekrümmte und auf die Rückseite (4d) der Umfangswand (4) gerichtete Form besitzen.

5. Karren nach einem der vorstehenden Ansprüche, **dadurch gekennzeichnet, dass** die Tablettstruktur (10) einen Griff (12) in Form eines Schlitzes umfasst, der in der Tablettstruktur (10) angebracht und abgemessen ist, um die Einführung in den Schlitz von mehreren Fingern einer Hand zu ermöglichen, um einem Bediener zu ermöglichen, den Karren (1) durch den Schlitz zu greifen.

6. Karren nach einem der vorstehenden Ansprüche, **dadurch gekennzeichnet, dass** sich die zwei Tragarme (8, 9) an die Wand (4) auf der Ebene von zwei Wanderweiterungen (21, 22) verbinden, die nach oben hervorspringen, vorzugsweise sind die Wanderweiterungen (21, 22) in den zwei lateralen Seiten (4b, 4c) angeordnet.

7. Karren nach Anspruch 6, **dadurch gekennzeichnet, dass** sich die Tragarme (8, 9) in den Sockel (2) auf der Ebene der Wanderweiterungen (21, 22) einfügen oder sich daran befestigen, vorzugsweise weisen die Tragarme (8, 9) und die Wanderweiterungen (21, 22) komplementäre dreidimensionale Strukturen (27, 27') auf.

8. Karren nach einem der Ansprüche 6 oder 7, **dadurch gekennzeichnet, dass** die Tragarme (8, 9) und die Wanderweiterungen (21, 22) Führungsschienen (31) und Gleitschienen umfassen, die zusammenarbeiten, um die Montage der Tragarme (8, 9) auf den Wanderweiterungen (21, 22) sicherzustellen.

9. Karren nach einem der Ansprüche 6, 7 oder 8, **dadurch gekennzeichnet, dass** die Tragarme (8, 9) an den Wanderweiterungen (21, 22) mit Hilfe eines Zwischenhalteteils (32) befestigt sind.

10. Karren nach einem der vorstehenden Ansprüche, **dadurch gekennzeichnet, dass** die Tablettstruktur (10) weiter mindestens eine Einrastvorrichtung (20) umfasst, vorzugsweise mehrere Einrastvorrichtungen, die insbesondere für das Einrasten eines Beatmungsballons (14) angepasst und entworfen sind.

11. Karren nach einem der vorstehenden Ansprüche, **dadurch gekennzeichnet, dass** der Sockel (2), der Boden, die Wand (4) aus einem einzigen Teil geformt sind, vorzugsweise bestehen sie aus polymerem Material.

12. Karren nach einem der vorstehenden Ansprüche, **dadurch gekennzeichnet, dass** er eine Befestigungsvorrichtung (18) einschließt, die ausgelegt und entworfen ist, um die Gasflasche (7) in der Aufnahme (5) zu halten, insbesondere eine medizinische Gasflasche, ausgewählt aus N₂O, O₂ und den Gemischen O₂/N₂O..

13. Verwendung eines Karrens (1) nach einem der vorstehenden Ansprüche in einem Krankenhausgebäude, um mindestens eine Gasflasche und eventuell eine medizinische Einrichtung (25) zu transportieren, die auf der Tablettstruktur (10) angeordnet ist, insbesondere ein Oximeter.

## Claims

1. Mobile cart (1) comprising:
- a base (2) with a bottom and provided with a plurality of wheels (3),
- the base (2) being topped by a peripheral wall (4) projecting upwards and conformed to delimit an inner volume (6) sized to form a housing able to receive a gas cylinder (7) of cylindrical shape, said gas cylinder (7) resting on said bottom, and
- two carrier arms (8, 9) projecting upwards, the carrier arms (8, 9) being secured by a first end (8a, 9a) to one of the side faces (4b, 4c) of the peripheral wall (4) and by a second end (8b, 9b) to a plate structure (10) topping the cart (1),
**characterised in that**:
- said peripheral wall (4) comprising a front face (4a) located opposite the rear face (4d) provided with the opening (5), and two side faces (4b, 4c) located opposite one another, with respect to the internal volume (6), the front face (4a) and the two side faces (4b, 4c) forming a compartment for a cylinder with an internal volume (6) and a U-shaped or near U-shaped cross-section, considered at the opening (5), the peripheral wall (4) having an opening (5) arranged in the rear face (4d) enabling the introduction of the gas cylinder (7) in said internal volume (6) and its positioning on the bottom of the base (2), and
- the cart (1) is formed by 2 sub-units connecting to one another and comprising:
• a first sub-unit comprising at least the base (2), the wheels (3), the bottom of the base (2) and the peripheral wall (4), and
• a second sub-unit comprising at least the carrier arms (8, 9) and the plate structure (10).

2. Cart according to the preceding claim, **characterised in that** one storage compartment (11) is arranged under the plate structure (10), said compartment (11) comprises one or more openings (13) providing access to an internal space.

3. Cart according to one of the preceding claims, **characterised in that** the plate structure (10) is flat and horizontal or near-horizontal, when the wheels of the cart are placed on a horizontal or near-horizontal floor.

4. Cart according to one of the preceding claims, **characterised in that** said side carrier arms (8, 9) have a curved elongated shape oriented towards the rear face (4d) of the peripheral wall (4).

5. Cart according to one of the preceding claims, **characterised in that** the plate structure (10) comprises a handle (12) in the form of a slot arranged in said plate structure (10) and sized to enable the introduction in said slot of a plurality of fingers of a hand, thereby enabling an operator to grip the cart (1) by said slot.

6. Cart according to one of the preceding claims, **characterised in that** the two carrier arms (8, 9) are connected to the wall (4) at two wall expansions (21, 22) protruding upwards, preferably said wall expansions (21, 22) are arranged in the two side faces (4b, 4c).

7. Cart according to claim 6, **characterised in that** the carrier arms (8, 9) are nested in or secured to the base (2) at the wall expansions (21, 22), preferably said carrier arms (8, 9) and said wall expansions (21, 22) have complementary three-dimensional structures (27, 27').

8. Cart according to one of claims 6 or 7, **characterised in that** the carrier arms (8, 9) and the wall expansions (21, 22) comprise guide rails (31) and sliders cooperating with one another to allow the mounting of said carrier arms (8, 9) on the wall expansions (21, 22).

9. Cart according to one of claims 6, 7 or 8, **characterised in that** the carrier arms (8, 9) are secured to the wall expansions (21, 22) by an intermediate retaining part (32).

10. Cart according to one of the preceding claims, **characterised in that** the plate structure (10) further comprises at least one hooking device (20), preferably a plurality of hooking devices, in particular suitable and designed for hooking a bag valve mask (14).

11. Cart according to one of the preceding claims, **characterised in that** the base (2), the bottom and the wall (4) are moulded in a single part, preferably made of a polymer material.

12. Cart according to one of the preceding claims, **characterised in that** it comprises an attachment device (18) suitable and designed to maintain the gas cylinder (7) inside the housing (5), in particular a cylinder containing a medical gas selected from N₂O, O₂ and O₂/N₂O mixtures.

13. Use of a cart (1) according to one of the preceding claims within a healthcare establishment to transport at least one gas cylinder, and possibly a medical apparatus (25) arranged on the plate structure (10), in particular an oximeter.
